**Europäisches Patentamt**

**European Patent Office**

**Office européen des brevets**

(19)

(11) Veröffentlichungsnummer : **0 100 985**
**B1**

(12) **EUROPÄISCHE PATENTSCHRIFT**

(45) Veröffentlichungstag der Patentschrift :
**08.07.87**

(21) Anmeldenummer : **83107543.7**

(22) Anmeldetag : **01.08.83**

(51) Int. Cl.⁴ : **C 07 K 7/10**, C 07 K 1/12,
A 61 K 37/02, A 61 K 37/64

(54) **Elastaseinhibitoren, Verfahren zu ihrer Herstellung sowie diese enthaltende Arzneimittel.**

(30) Priorität : **14.08.82 DE 3230275**

(43) Veröffentlichungstag der Anmeldung :
**22.02.84 Patentblatt 84/08**

(45) Bekanntmachung des Hinweises auf die Patenterteilung : **08.07.87 Patentblatt 87/28**

(84) Benannte Vertragsstaaten :
**CH DE FR GB IT LI**

(56) Entgegenhaltungen :
**Keine Entgegenhaltungen**

(73) Patentinhaber : **BAYER AG**
**Konzernverwaltung RP Patentabteilung**
**D-5090 Leverkusen 1 Bayerwerk (DE)**

(72) Erfinder : **Hochstrasser, Karl, Prof. Dr.**
**Blumenweg 8**
**D-8031 Seefeld-Oberalting (DE)**
Erfinder : **Wachter, Elmar, Dr.**
**Gustav-Schiefer-Strasse 3b**
**D-8000 München 50 (DE)**

## Beschreibung

Die vorliegende Erfindung betrifft neue Inhibitoren aus Rinderserum für Pankreas- und Granulozyten-Elastase, die im folgenden als BI-8-E und BI-8-E⁺ (Bovine Inhibitoren mit Mr 8 000 für Elastase) bezeichnet werden, sowie ein Verfahren zu ihrer Herstellung und diese enthaltende Arzneimittel.

Aus dem Inter-α-trypsininhibitor, abgekürzt ITI, des Rindes wird durch einen oder mehrere enzymatische Reaktionsschritte neben einem strukturhomologen Trypsin-Chymotrypsin-Inhibitor, der im folgenden BI-8-T (Bovine Inhibitor mit Mr 8 000 für Trypsin) genannt wird, der erfindungsgemäße Elastaseinhibitor BI-8-E freigesetzt und durch chromatographische Verfahren rein dargestellt. Der BI-8-E ist ein Glykoprotein bei dem über die Seitenkette des Asn — in Position 24 der Peptidkette — ein aus N-Acetyl-neuraminsäure-, N-Acetylglucosamin-, Galaktose- und Mannose-resten aufgebautes Kohlenhydrat N-glycosidisch gebunden ist. Durch Abspalten des Glycosidrestes nach literaturbekannten Verfahren erhält man aus dem BI-8-E einen erfindungsgemäßen modifizierten Inhibitor, der im folgenden als BI-8-E⁺ bezeichnet wird. Beide Inhibitoren BI-8-E und BI-8-E⁺ haben, wie aus Tab. 1 ersichtlich, identische Hemmspektren. BI-8-E⁺ hat die folgende Struktur :

Lys-Ala-Asp-Ser-Cys-Gln-Leu-Asp-Tyr-Ser-Gln-Gly-Pro-Cys-Leu-Gly-Leu-Phe-Lys-Arg-Tyr-Phe-Tyr-Asn-Gly-Thr-Ser-Met-Ala-Cys-Glu-Thr-Phe-Leu-Tyr-Gly-Gly-Cys-Met-Gly-Asn-Leu-Asn-Asn-Phe-Leu-Ser-Gln-Lys-Glu-Cys-Leu-Glu-Thr-Cys-Arg.

Diese Struktur ist der von BPTI (Basic Pancreatic Trypsin Inhibitor, Kunitz-Inhibitor) aus Rinderorganen mit einem Lysinrest im aktiven Zentrum Position 15 bzw. P1 nach der Nomenklatur von Schechter und Berger [I. Schechter und A. Berger, Biochem. Biophys. Res. Commun. 27, 157-162 (1967)] sehr ähnlich, und der Inhibitor wird deshalb als ein Inhibitor vom Kunitz-Typ bezeichnet.

Der die Spezifität der erfindungsgemäßen Kunitz-Inhibitoren bedingende Aminosäurerest in Position 15 bzw. P1 ist Leucin. Die Inhibitoren sind potente Hemmer von Pankreas- und Granulozyten-Elastase, Chymotrypsin und Kathepsin G. Sie sind deshalb erfindungsgemäß verwendbar als Arzneimittel zur Therapie von Erkrankungen, die ausgelöst werden entweder durch eine Überproduktion dieser Proteinasen in Folge einer erhöhten Freisetzung aus den Zymogenen bzw. einer Ausschüttung beim Zellzerfall oder aber durch einen Mangel bzw. einen Fehlen von natürlichen körpereigenen Inhibitoren der Enzyme in Organen und Gewebsflüssigkeiten. Erkrankungen mit derartiger Äthiologie sind die verschiedenen Formen des Schocks, posttraumatische oder postoperative Komplikationen, Störungen der Blutgerinnung, akute und chronische Entzündungsreaktionen, insbesondere auch chronische Entzündungsreaktionen mit nekrotischen und degenerativen Bindegewebsschädigungen, wie Pankreatitis, sowie durch Immunkomplexe bedingte Vasculitiden, Glomerulonephritiden, rheumatoide Arthritis und andere Collagenosen sowie durch stoffwechselbedingte Ablagerungen verursachte Arthritiden (Gicht), aber auch degenerative Veränderungen der elastischen Elemente von Gefäßwänden (Atherosklerose) oder der Lunge (Lungenemphysem).

Es ist bereits bekannt geworden, daß BPTI [H. Kraut, E.K. Frey, E. Werle, Z. Physiol. Chem. 189, 97 (1930)], auch als Kunitz-Inhibitor bezeichnet [M. Kunitz, H. H. Northrop, J. Gen. Physiol. 19, 991 (1936)], eine Reihe von physiologisch bedeutsamen Enzymen, wie z. B. Kininogenine (Kininogenasen), Plasmin, Chymotrypsin und Trypsin hemmt [E. Werle in W. Brendel, G. Haberland : Neue Aspekte der Trasylol-Therapie 5, 9, F.K. Schattauer-Verlag Stuttgart-New York 1972 ; H. Fritz, H. Tschesche, L.J. Greene, E. Truscheit (Hrsg.) : Proteinase Inhibitors (Bayer Symposium V), Proc. 2nd International Research Conference, Springer-Verlag Berlin-Heidelberg-New York 1974] und als Aprotinin (generic name) zur Therapie und Prophylaxe von Schockzuständen sowie zur Prophylaxe postoperativer und posttraumatischer Komplikationen eingesetzt wird.

Es ist weiterhin bekannt, daß bei der Enteiweißung von Rinderserum mit Perchlorsäure analog wie bei der Enteiweißung von Humanserum der säurestabile, physiologische ITI mit einem Molekulargewicht (Mr) von 30 000 erhalten wird [E. Wachter, K. Deppner, K. Hochstraßer, K. Lempart und R. Geiger, FEBS Letters (1980) 119, 58 bis 62], der im folgenden als BI-30 bezeichnet wird. Der Perchlorsäureüberstand enthält jedoch anders als beim Humansystem einen weiteren Trypsininhibitor mit Mr 8 000, der auch andere Proteinasen, nicht aber Elastasen, hemmt und ebenfalls zur Klasse der Kunitz-Inhibitoren gehört. Er stammt nicht aus ITI und wird im folgenden BI-8⁺ genannt.

Es ist weiterhin bekannt, daß aus dem Perchlorsäurepräzipitat durch partielle enzymatische Spaltung mit überschüssigen Proteinasen wie Trypsin, Plasmin, Elastase oder Kallikrein in schwach alkalischer Lösung, wie von Hochstrasser und Mitarbeitern [K. Hochstrasser, G. Bretzel, H. Feuth, W. Hilla und K. Lempart, H.S. Z. Physiol. Chem. 357, 153-162 (1976)] für das humane System beschrieben, weitere Inhibitoren vom Kunitz-Typ freigesetzt werden, die wie im Humansystem dem ITI entstammen.

Bei dieser limitierten Proteolyse wird, wie aus dem Humansystem bekannt [E. Wachter, K. Hochstrasser, G. Bretzel und S. Heindl, H.S. Z. Physiol. Chemin. 360, 1297-1303 (1979)], ein polyvalenter Inhibitor mit Mr 14 000 erhalten, der als BI-14 bezeichnet wird, und in dem zwei Inhibitoren vom Kunitz-Typ mit unterschiedlichen Spezifitäten über eine Arg-Thr-Bindung kovalent verknüpft sind.

Es wurde nun gefunden, daß diese Arg-Thr-Bindung durch längeres Behandeln des BI-14 mit Proteinasen in schwach alkalischer Lösung selektiv so gespalten werden kann, daß die volle inhibitorische Wirkung beider Domänen erhalten bleibt.

Für diese Proteolyse geeignete Proteinasen sind erfindungsgemäß Kallikreine aus Harn oder Pankreas, Plasmin und insbesondere Trypsin, jedoch auch Proteinasen aus Pflanzen, Pilzen oder Bakterien. Die Proteinasen können vorteilhaft immobilisiert, gebunden auf festen, inerten Trägern, eingesetzt werden. Bei der Verwendung von immobilisiertem Trypsin, vorzugsweise Trypsin-Sepharose, ist ein Zusatz des löslichen Enzyms vorteilhaft. Die Bindung des antitryptisch aktiven BI-14 an die Trypsin-Sepharose erfolgt auch aus verdünnten Lösungen vollständig, und die Proteolyse mit überschüssigem gebundenem oder zugesetztem löslichem Trypsin verläuft am Träger, wobei nur diejenigen Inhibitoren in die überstehende Lösung gelangen, die Trypsin nicht hemmen.

Für die Spaltung werden die Proteinasen, insbesondere diejenigen, die durch BI-14 gehemmt werden, in einem molaren Überschuß, bezogen auf die Inhibitoren, eingesetzt. Dieser Überschuß kann 10-400 % betragen ; vorzugsweise liegt er zwischen 150 und 300 %. Die Reaktionen werden in gepufferten Lösungen mit pH-Werten von 6,5-10, insbesondere 7,5-8,5 durchgeführt. Geeignete Puffer werden hergestellt unter Verwendung von Tris-(hydroxymethyl)-aminomethan, Triethanolamin, Alkali-Boraten und -Phoshaten oder auch Alkali-Carbonaten. Die Puffer können gegebenenfalls auch organische Lösungsmittel, wie Dimethylformamid, Dimethylsulfoxid oder Hexamethylphosphorsäuretriamid und/oder Salze als Zusätze enthalten. Die Temperatur der Reaktionsansätze wird während der Proteolyse vorzugsweise bei 37 °C gehalten. Man kann die Spaltung aber auch bei tieferen oder bei höheren Temperaturen vornehmen. Die Umsetzungsdauer beträgt 1-24 Std., vorzugsweise 1-10 Std. Die Spaltung verläuft nicht vollständig. Der Reaktionsansatz enthält neben unumgesetztem BI-14 die beiden Spaltprodukte BI-8-T und BI-8-E sowie einen weiteren Elastase-Chymotrypsininhibitor vom Mr 14 000, dessen antitryptisches Zentrum offenbar modifiziert ist, und der im folgenden als BI-14$^+$ bezeichnet wird.

Bevorzugtes Ausgangsmaterial für die Gewinnung des erfindungsgemäßen Elastase-Inhibitors BI-8-E ist der Tandem-Kunitz-Inhibitor BI-14 aus dem Rinder-ITI. Dieser säurestabile, polyvalente Inhibitor wird, wie bereits erwähnt, durch limitierte Proteolyse des bei der Enteiweissung anfallenden Präzipitats erhalten. Relativ geringe Mengen an BI-14 und seiner Vorstufe, dem BI-30, können zusätzlich, wie bereits bekannt, aus dem Überstand der Perchlorsäurefällung isoliert werden [E. Wachter, K. Deppner, K. Hochstrasser, K. Lempart und R. Geiger, FEBS Letters (1980) 119, 58-62].

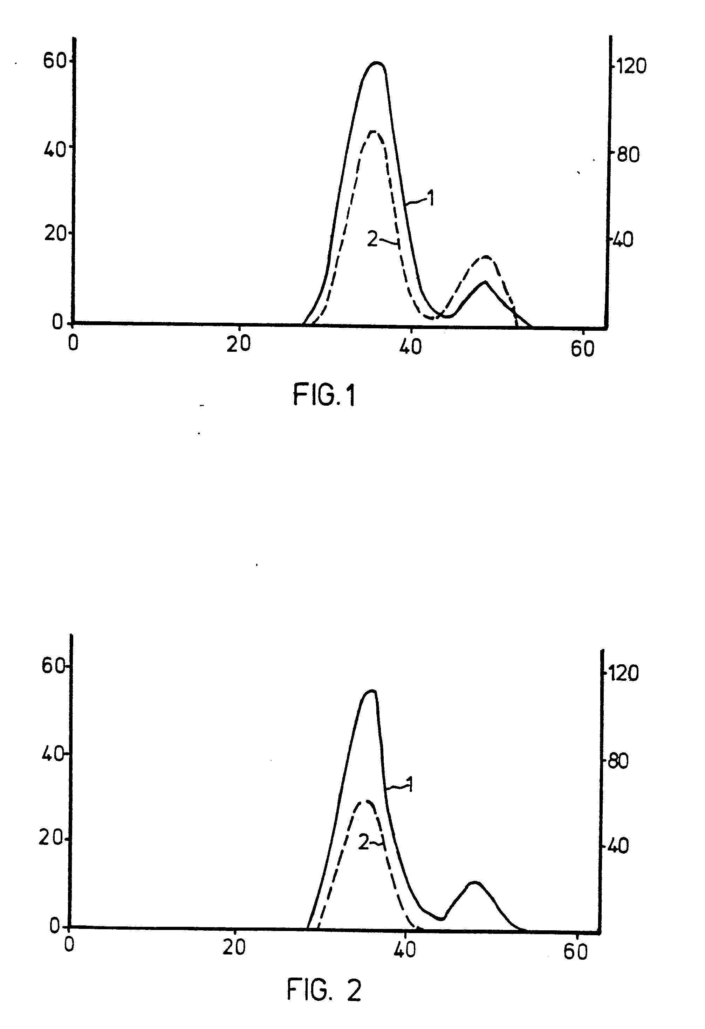

Erfindungsgemäß kann die Gewinnung von BI-8-E auch ausgehend vom BI-30 durch vollständige Proteolyse durch geführt werden unter Verwendung der bereits aufgeführten Enzyme, insbesondere aber von Trypsin. Es ist ebenso möglich, das Gemisch von BI-14 und BI-30 zu verwenden oder aber das Rinderserum selbst. Naturgemäß ist im letzteren Falle für die Spaltung eine erheblich größere Menge an Enzym nötig, da auch noch die säurelabilen Inhibitoren zuvor abgesättigt bzw. komplexiert werden müssen. In allen Fällen muß nun der gewünschte BI-8-E von den inhibitorisch inaktiven Begleitstoffen sowie den inhibitorisch aktiven Substanzen wie BI-14, BI-14$^+$, BI-8-T und bei Verwendung von Rinderserum oder der bei der Enteiweissung anfallenden Inhibitorfraktion bzw. rohem BI-14, der über eine reversible Komplexierung mit einem Trypsin beladenen Träger erhalten wurde, zusätzlich BI-8$^+$ abgetrennt werden. In Abb. 1 ist das Elutionsprofil einer mit dem BI-14 Trypsinolyseansatz beschickten Sephadex G-75$^{(R)}$-Säule wiedergeben — Säulendimensionen 3 × 200 cm ; Elutionsmittel 0,05 M Boratpuffer pH 8,0, der 0,2 M an Natriumchlorid war ; es wurden Fraktionen zu jeweils 24 ml aufgefangen —. Auf der Abszisse sind die Nummern der Fraktionen angegeben, auf der Ordinate links die antitryptische und rechts die Anti-Elastase-Aktivität der Eluate. Die Angaben zur Hemmung von Trypsin bzw. Elastase erfolgen in Milli-Inhibitor-Einheiten/ml Eluat — mIU/ml —. Die Hemmung des Trypsin wurde ermittelt, wie unter 2.d. beschrieben, die Hemmung von Schweinepankreas-Elastase, wie unter 2.a. angegeben. Die Kurve 1 gilt für die Elastasehemmung, während Kurve 2 für die Trypsinhemmung zutrifft.

Für die Abtrennung der trypsininhibitorisch aktiven Substanzen eignet sich insbesondere die Komplexierung mit trägergebundenem Trypsin. Dazu wird das für die Spaltung verwendete Enzym zuerst vorzugsweise durch Fällung mit Perchlorsäure und Zentrifugation oder Filtration abgetrennt. Nach dem Neutralisieren und Einstellen eines pH-Wertes zwischen 7,5 und 10 wird die die Inhibitoren enthaltende Lösung mit dem Trypsin-beladenen Trägern versetzt oder über diesen Träger filtriert.

BI-8-E komplexiert nicht mit dem Trypsin und BI-14$^+$ hat nur eine geringe Affinität zum Trypsin. Beide werden im Durchlauf bzw. bei Nachwaschen im Filtrat erhalten. Die Trennung von BI-14$^+$ und BI-8-E wird vorzugsweise durch Filtrieren des Gemisches über eine Molekularsiebsäule durchgeführt. Geeignete Molekularsiebe sind quervernetzte Dextrane, wie Sephadex G-50$^{(R)}$, Sephadex G-75$^{(R)}$ oder Sephadex G-100$^{(R)}$, Bio-Gel P-30$^{(R)}$, Biol-Gel P-60$^{(R)}$ oder Bio-Gel P-100$^{(R)}$. Als Lösungsmittel für die Gelfiltration eignen sich insbesondere leichtflüchtige verdünnte Säuren wie Essigsäure oder flüchtige Puffer- bzw. Salzlösungen wie Ammoniumformiatlösungen, Ammoniumbicarbonat- oder Ammoniumacetatlösungen. Zur weiteren Reinigung von BI-8-E wird der Inhibitor an einem mit Chymotrypsin beladenen Träger vorzugsweise an α-Chymotrypsin-Sepharose aus neutraler oder schwach alkalischer Lösung unter Komplexbildung adsorbiert. Nach dem Auswaschen der Verunreinigungen wird der Komplex durch Behandlung des beladenen Affinitätsträgers mit sauren Puffern in an sich bekannter Weise im Batch oder in einer Säule unter Freisetzung des Inhibitors zerlegt. Führt man die Zerlegung des Komplexes in einer Säule durch, so hat man gleichzeitig den Vorteil, daß die Elution in einem relativ kleinen Volumen erfolgt. Die weitere Konzentrierung ist möglich durch eine Ultrafiltration, wobei gleichzeitig die Salze abgetrennt werden. Die Abtrennung der Salze ist jedoch auch durch eine Gelfiltration des Konzentrates über Molekularsiebsäulen in der oben beschriebenen Weise möglich.

Alternativ läßt sich der BI-8-E auch durch Adsorption an Concanavalin-A-Sepharose [R] reinigen. Vor der Desorption mit einer Lösung von α-Methylglucosid oder α-Methylmannosid werden die Verunreinigen mit Wasser und/oder Pufferlösungen ausgewaschen. Der so erhaltene BI-8-E kann nach dem Entsalzen durch Ultrafiltration mit einer Amicon UM-2-Membran oder Filtration über eine Molekularsiebsäule durch Gefriertrocknen isoliert werden.

Für die Isolierung von BI-8-E ist es besonders vorteilhaft, den Durchlauf bzw. das Filtrat der Trypsin-Säule, die BI-14$^+$ und BI-8-E enthalten, ohne weitere Manipulationen an einen mit Chymotrypsin belandenen Träger zu binden. Die Komplexierung des Inhibitors mit dem trägergebundenen Enzym kann im Batch oder durch Filtration über eine mit dem Affinitätsträger gefüllte Säule durchgeführt werden, wobei die Bindung aus neutraler oder schwach alkalischer Lösung erfolgt.

Nach dem Auswaschen der Verunreinigungen werden die Inhibitoren durch Ansäuern mit verdünnten Säuren in vorzugsweise salzhaltigen Lösungen aus den Komplexen freigesetzt. Geeignete Säuren sind insbesondere Salzsäure oder Ameisensäure und Essigsäure. Als Salze werden vorzugsweise Natriumchlorid oder Kaliumchlorid verwendet, doch eignen sich auch andere anorganische Salze sowie denaturierende Agentien wie Lithiumchlorid, Guanidiniumchlorid oder Harnstoff. Die Freisetzung kann entweder im Batch oder in einer Säule durchgeführt werden. Die Isolierung von BI-8-E wird schließlich nach dem Neutralisieren und Einengen durch Gelfiltration über geeignete und bereits aufgeführte Molekularsiebe mit den ebenfalls schon erwähnten Elutionsmitteln vorgenommen. Bei diesem Verfahren werden gleichzeitig die Salze abgetrennt. Das BI-8-E läßt sich, wie mehrfach erwähnt, durch Gefriertrocknung isolieren. In Abb. 2 ist das Elutionsprofil der Sephadex G-75 Säule — 3 × 200 cm — wiedergegeben. Die Säule wurde mit 0,05 M Boratpuffer pH 8,0, 0,2 M an Natriumchlorid eluiert und die Eluate in Fraktionen zu je 24 ml aufgefangen. In den Eluaten wurden die spezifische Trypsinhemmung, wie unter 2.d. beschrieben, und die spezifische Hemmung der Schweinepankreas-Elastase, wie unter 2.a.α. angegeben, bestimmt und die Inhibitor-Einheiten — eine IU (Inhibitor-Einheit) entspricht der Menge Inhibitor die 2 Enzymeinheiten zu 50 % hemmt — ermittelt. Auf der Ordinate sind (Abb. 2) links die Trypsinhemmung — Kurve 2 — und rechts die Elastasehemmung — Kurve 1 — in mIU/ml angegeben und auf der Abszisse die Fraktionsnummern.

Es ist aber auch möglich, aus dem rohen Spaltungsgemisch die kohlenhydrathaltigen Inhibitoren zunächst in der schon beschriebenen Weise an eine Concanavalin-A-Säule zu absorbieren und, wie ebenfalls bereits beschrieben, nach dem Auswaschen der Begleitsubstanzen mit einer α-Methylglycosidlösung zu desorbieren. Die in diesem Desorbat enthaltenen Inhibitoren können dann durch Gelfiltration an Molekularsiebsäulen getrennt werden, in der schon mehrfach beschriebenen Weise, wobei gleichzeitig das α-Methylglycosid und gegebenenfalls in der Lösung enthaltene Salze abgetrennt werden.

Erfindungsgemäß kann der kohlenhydratfreie BI-8-E, im folgenden als BI-8-E$^+$ bezeichnet, ausgehend vom Lyophilisat oder auch von der Lösung, die gegebenenfalls eingeengt wird, erhalten werden, indem man die Kohlenhydrate in an sich bekannter Weise mit Säure abspaltet. Besonders vorteilhaft ist die Verwendung von 60-90 % Ameisensäure bei Temperaturen von 40-70 °C, vorzugsweise bei 56 °C.

Es ist jedoch auch möglich, andere Säuren, wie M Schwefelsäure oder M Salzsäure, bei erhöhten Temperaturen zwischen 50 und 100 °C, vorzugsweise 90 °C, einzusetzen. Die Abtrennung der Hydrolyseprodukte und der Säure vom BI-8-E$^+$ gelingt nach dem Neutralisieren entweder durch Ultrafiltration unter Verwendung von Filtern definierter Porengröße, wie beispielsweise Amicon UM-2-Membranen, oder aber nach dem Einengen der Lösung durch Filtration über eine mit einem geeigneten Füllmaterial gefüllte Molekularsiebsäule.

Zur Isolierung der erfindungsgemäßen Inhibitoren können auch mit Antikörpern, insbesondere monoklonalen Antikörpern beladene Affinitätsträger verwendet werden.

Die erfindungsgemäßen Elastaseinhibitoren BI-8-E und BI-8-E$^+$ haben, wie schon eingangs ausgeführt, einen Leucin-Rest in der für die Spezifität des Inhibitors entscheidenden Position P1 der Peptidkette. Nach den Ergebnissen der Arbeitsgruppen von Powers und Zimmerman [M. Zimmerman u. B. M. Ashe, Biochim. Biophys. Acta 480, 241 (1977) ; J.C. Powers, B.F. Gupton, A.D. Harley, N. Nishino und R.C. Whitley, Biochim. Biophys. Acta 485, 156 (1977) ; M. Castillo, K. Nakajima, M. Zimmerman und J.C. Powers, Analyt. Biochem. 99, 53 (1979) u. K. Nakajima, J.C. Powers, B.M. Ashe und M. Zimmerman, J. Biol. Chem. 254, 4027 (1979)] bei Untersuchungen über die Affinität von synthetischen Substraten und Inhibitoren für Granulozytenelastase, Pankreaselastase, Chymotrypsin und Kathepsin G überrascht die gute Hemmwirkung des Leu$^{15}$-Inhibitors für all diese Enzyme.

Die erfindungsgemäßen Inhibitoren haben gegenüber dem BPTI überlegene biologische Eigenschaften. Von besonderem Vorteil sind ihre inhibitorischen Wirkungen auf die Elastasen aus Pankreas und Granulozyten sowie auf Kathepsin G, die neue therapeutische Einsatzmöglichkeiten eröffnen. Pankreas-Elastase spielt eine wichtige Rolle bei der Pankreatitis [M. C. Geokas, H. Rinderknecht, V. Swanson, B.P. Vitron und B.J. Haverback, Clin. Res. 16, 285 (1968)] ; Serumelastase bei der Atherosklerose [U. Butturini u. M. Langen, Klin. Wochenschr. 49, (1962)] und Granulozytenelastase bei akuten und chronischen Entzündungen mit Bindegewebsschädigung [A. Janoff, Amer. J. Pathol. 68, 579 (1972)], bei Gefäßwandschädigungen [A. Janoff und J.D. Zeligs, Science 161, 702 (1968)] sowie bei nekrotisierenden Erkrankungen und Degeneration von Lungengewebe, z. B. beim Emphysem [G.M. Turino, R.M. Senior, B.D. Garg, S. Keller, M.M. Levi und I. Mandl, Science 165, 709 (1969) ; H. E. Evans, M.M. Levi und I. Mandl, Amer. Rev. Respir. Dis. 101, 359 (1970) sowie A. Janoff, R.A. Sandhaus, V.D. Hospelhorn und R. Rosenberg, Proc. Soc. Exptl. Biol. Med. 140,. 516 (1972)]. Ebenso wichtig ist die Rolle von lysosomalen Enzymen und insbesondere der Granulozytenelastase bei immunologisch bedingten Entzündungsreaktionen [M. Koono, M. Muto und H. Hayashi, Tohoku J. Exptl. Med. 94, 231 (1968)], z. B. der rheumatoiden Arthritis [G. Weissmann und J. Spilberg, Arthritis Rheumat. 11, 162 (1968)].

Ein weiterer Vorteil der erfindungsgemäßen Inhibitoren ist ihre geringe Antigenität und Immunogenität. So reagiert zwar BI-14 mit Anti-ITI-Antikörpern, nicht aber BI-8-E und BI-8-E$^+$.

Die Bereitstellung der neuen erfindungsgemäßen Inhibitoren stellt somit eine Bereicherung der Pharmazie dar. Die erfindungsgemäßen aus Rinderserum gewonnenen Inhibitoren sind neu. Sie lassen sich durch chemische, physikalich-chemische, biochemische sowie biologische Eigenschaften charakterisieren und gegenüber bekannten Substanzen abgrenzen. Es wurden folgende Kriterien herangezogen :

1. Bestimmung der Sequenz der Aminosäuren

300 mg BI-8-E$^+$ wurden reduziert und die Mercaptogruppen der Cysteinreste carboxymethyliert, wie von Crestfield und Mitarbeitern beschrieben [A.M. Crestfield, S. Moore und W.H. Stein, J. Biol. chem. 238, 622-627 (1963)]. Nach dem Abtrennen der überschüssigen Reagentien durch Gelfiltration über. Bio-Gel P-2 wurden aliquote Teile des Inhibitors mit Chymotrypsin und Trypsin, im letzten Fall zusätzlich auch nach Citraconylierung in an sich bekannter Weise [K. Hochstrasser und E. Wachter, H.S. Z. Physiol. Chem. 360, 1287-1296 (1979)], enzymatisch abgebaut. Die so erhaltenen Substanzgemische wurden, wie von Hochstrasser und Wachter in obiger Arbeit beschrieben, durch Ionenaustausch- und Gelchromatographie in ihre Komponenten aufgetrennt. Die Aminosäuresequenzen der einzelnen Komponenten wurden durch Edman-Abbau in einem Festphasensequentiator nach R.A. Laursen [R.A. Laursen, Europ. J. Biochem. 20, 80-91 (1971)] ermittelt.

Durch Aneinanderreihen der entsprechenden Partialsequenzen wurde die Gesamtsequenz von BI-8-E$^+$ bestimmt zu :

Lys-Ala-Asp-Ser-Cys-Gln-Leu-Asp-Tyr-Ser-Gln-Gly-Pro-Cys-Leu-Gly-Leu-Phe-Lys-Arg-Tyr-Phe-Tyr-Asn-Gly-Thr-Ser-Met-Ala-Cys-Glu-Thr-Phe-Leu-Tyr-Gly-Gly-Cys-Met-Gly-Asn-Leu-Asn-Asn-Phe-Leu-Ser-Gln-Lys-Glu-Cys-Leu-Glu-Thr-Cys-Arg.

2. Proteasen-Inhibitionsspektrum

a) Elastase-Inhibition

α) Pankreas-Elastase-Inhibition

Für die Hemmversuche mit den erfindungsgemäßen Inhibitoren wurde kristallisierte Pankreaselastase (Schwein) der Fa. Nutritional Biochemicals Corp. verwendet. Als Substrat wurde Succinyl-L-alanyl-L-alanyl-L-alanin-p-nitroanilid [J. Bieth, B. Spiess und C.G. Wermuth, Biochm. Med. 11, 350 (1974)] eingesetzt. Die Spaltung wurde durch kontinuierliche Messung der Extinktion des freigesetzten p-Nitroanilins bei 405 nm bestimmt. Um maximale Komplexbildung sicherzustellen, wurden Enzym und Inhibitor vor der Zugabe des Substrats für 15 Min. vorinkubiert.

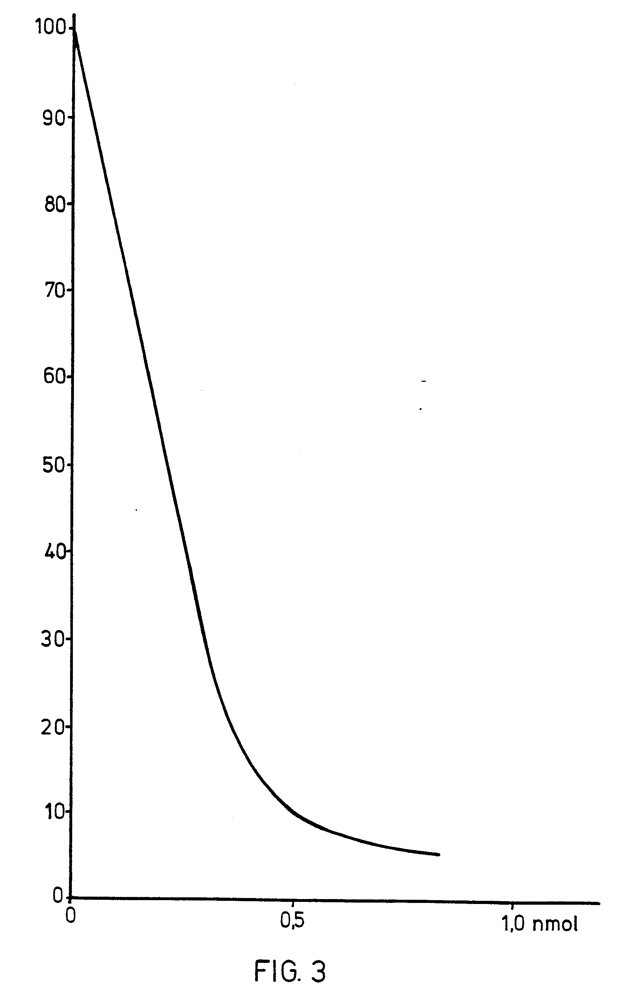

In Tab. 1 sind semiquantitative Angaben über die Inhibition des Enzyms für die hier relevanten Inhibitoren zusammengestellt. Abb. 3 zeigt die Titrationskurve von Pankreaselastase (Schwein) mit dem erfindungsgemäßen BI-8-E. Auf der Ordinate ist die relative Aktivität der Elastase, auf der Abszisse die Menge des Inhibitors (in nmol) aufgetragen.

β) Granulozyten-Elastase-Inhibition

Als Substrat wurde Succinyl-L-alanyl-L-alanyl-L-alanin-p-nitroanilid [J. Bieth, B. Spiess und C.G. Wermuth, Biochem. Med. 11, 350 (1974)] verwendet. Angaben über die Hemmung von Granulozytenelastase für die hier relevanten Inhibitoren sind in Tabelle 1 aufgenommen.

b) Chymotrypsin-Inhibition

Die Aktivität von Chymotrypsin wurde nach W. Nagel, F. Willig, W. Peschke und F.H. Schmidt, H.S. Z. Physiol. Chem. 340, 1 (1965) photometrisch mit Succinyl-L-phenylalanin-p-nitroanilid als Substrat bestimmt und die Hydrolyse durch kontinuierliche Messung der Extinktion des freigesetzten p-Nitroanilins bei 405 nm bestimmt. Vor der Zugabe des Substrats wurden Enzym und Inhibitor im Testpuffer 15 Min. vorinkubiert.

Als weiteres Substrat wurde Succinyl-L-phenylalanin-β-naphthylester in dem von Schnabel [E. Schnabel, H.S. Z. Physiol. Chem. 362, 655-664 (1981)] beschriebenen kontinuierlichen Test eingesetzt. Vor der Zugabe des Substrats wurden Enzym und Inhibitor 10-15 Min. bei Raumtemperatur im Testpuffer vorinkubiert.

In Tabelle 1 sind Angaben zur Chymotrypsinhemmung für die hier relevanten Inhibitoren enthalten.

c) Kathepsin G-Inhibition

Die Aktivität von Kathepsin G wurde ebenfalls mit Succinyl-L-phenylalanin-β-naphthylester nach E. Schnabel [H.S.Z. Physiol. Chem. 362, 655-664 (1981)] bestimmt.

Tabelle 1 enthält Angaben zur inhibitorischen Aktivität der hier relevanten Inhibitoren.

d) Trypsin-Inhibitoren

Die Trysinaktivität wurde nach H. Fritz, I. Trautschold u. E. Werle [in Methoden der enzymatischen Analyse, Hrsg. H.W. Bergmeyer, 2. Aufl. Bd. 1, 1011 (1970)] mit Benzoyl-L-arginin-p-nitroanilid als Substrat bestimmt. Das freigesetzte p-Nitroanilin wurde spektrophotometrisch bei 405 nm gemessen. Enzym und Inhibitor wurden vor der Zugabe des Substrats 15 Min. vorinkubiert. In Tab. 1 finden sich Angaben zur Hemmbarkeit von Trypsin durch die hier relevanten Inhibitoren.

Es wurde weiterhin gefunden, daß die erfindungsgemäßen Inhibitoren in Modellen der akuten Entzündungsreaktion prophylaktisch und auch therapeutisch wirken. Die Entzündungsreaktionen werden auch dann signifikant gehemmt, wenn die Inhibitoren mehrere Stunden nach dem Setzen der Entzündungsnoxe verabreicht werden. Diese therapeutische Wirkung der Inhibitoren ist bedingt durch die große Verweildauer im Körper der Versuchstiere sowie das Hemmspektrum.

Versuchsanordnung zum Nachweis entzündungshemmender Wirkung bei der Ratte

a) Kaolin-induzierte Entzündungsreaktion

Die Entzündungsreaktion wurde durch intraplantare Injektion von 0,1 ml einer 10 %igen Kaolinsuspension in eine Hinterpfote von 130-160 g schweren Wistar-Ratten induziert. Die für die Behandlung der Entzündungsreaktion verwendeten erfindungsgemäßen Inhibitoren wurden in 0,9 %iger Natriumchloridlösung in einer Konzentration von 10-20 mg/ml gelöst. Die Behandlung der Versuchstiere erfolgte durch intraperitoneale, intramuskuläre, subkutane oder intravenöse Injektion von 0,5-1,0 ml Lösung der Inhibitoren entweder prophylaktisch, d. h. vor Setzen der Entzündungsnoxe, oder therapeutisch, d. h. nach Setzen der Entzündungsnoxe. Die Schwellung der entzündeten Pfote, die ein Mass für die Schwere der Entzündungsreaktion ist, wurde mit dem Antiphlogmeter nach Kemper [F. Kemper und G. Ameln, Z. ges. exp. Med. 131, 407-411 (1959)] zeitlich verfolgt.

Zur Ermittlung der Dosis-Wirkungsbeziehungen wurde der 4 Stunden nach Setzen der Entzündungsnoxe gemessene Wert verwendet.

Der Wirkungsvergleich zeigt, daß die neuen Inhibitoren dem BPTI in ihrer entzündungshemmenden Wirkung überlegen sind.

b) Aerosil-induzierte Entzündungsreaktion

Die Entzündungsreaktion wurde durch intraplantare Injektion von 0,1 ml einer 2 %igen Aerosilsuspension in eine Hinterpfote von 130-160 g schweren Wistar-Ratten induziert. Die für die Behandlung der Entzündungsreaktion verwendeten erfindungsgemäßen Inhibitoren wurden in 0,9 %iger Natriumchloridlösung in einer Konzentration von 10-20 mg/ml gelöst. Die Behandlung der Versuchstiere erfolgte durch intraperitoneale, subkutane oder intravenöse Injektion von 0,5-1,0 ml Lösung der Inhibitoren 15 Std. nach Setzen der Entzündungsnoxe. Die Schwellung der entzündeten Pfote, die ein Maß für die Schwere der Entzündungsreaktion ist, wurde mit dem Antiphlogmeter nach Kemper zeitlich verfolgt. Zur Ermittlung der Dosis-Wirkungsbeziehungen wurde der 21 Stundenwert nach Entzündungsinduktion (= 6 Std. nach Injektion der erfindungsgemäßen Inhibitoren) ermittelt.

Das Ergebnis der Therapieversuche mit den neuen Inhibitoren nach den Beispielen zeigt die Wirksamkeit der verwendeten Inhibitoren in diesem experimentellen Modell, in dem BPTI in gleicher Dosierung die Entzündungsreaktion nicht hemmt.

Die neuen erfindungsgemäßen aus dem Rinder-ITI hergestellten Inhibitoren können aufgrund ihrer biologischen Wirksamkeit insbesondere zur Behandlung folgender Krankheiten bzw. Krankheitserscheinungen verwendet werden :

1. Verschiedene Formen des Schocks, insbesondere Schocklunge und Endotoxinschock, posttraumatische und postoperative Komplikationen,

2. Störungen der Blutgerinnung,

3. akute und chronische Entzündungsreaktionen, insbesondere zur Therapie und Prophylaxe von Organschädigungen, wie beispielsweise Pankreatitis und strahleninduzierte Enteritis, immunkomplexbedingte Entzündungsreaktionen, wie Immunvasculitis, Glomerulonephritis und Arthritiden ; Kollagenosen, insbesondere rheumatoide Arthritis,

4. durch stoffwechselbedingte Ablagerungen verursachte Arthritiden (z. B. Gicht),
5. Degeneration der elastischen Bestandteile der Bindegewebsteile von Organen, wie bei der Atherosklerose oder dem Lungenemphysem,
6. strahleninduzierte Enteritis.

Die neuen Wirkstoffe können in bekannter Weise (analog BPTI) in die üblichen Formulierungen übergeführt werden.
Folgende Formulierungen sind dabei bevorzugt zu nennen :

1. Lösungen für parenterale Anwendung zur intravenösen, intramuskulären, subkutanen Injektion bzw. zur intraartikulären und intratumoralen Injektion,
2. Lösungen für intravenöse Dauerinfusion,
3. Lösungen zur Anwendung als Aerosole zur Inhalation,
4. Lösungen, Emulsionen, Salben, Pasten, Cremes, Lotions, Puder zur äußerlichen lokalen Anwendung,
5. Kombination mit anderen Hemmstoffen, deren Wirkungsspektrum sich gegenseitig ergänzt.

Die Konzentrationen der neuen Wirkstoffe in den erfindungsgemäße Formulierungen bewegen sich dabei in den Grenzen 0,01 bis 100 mg/ml Lösung, vorzugsweise zwischen 0,1 bis 10 mg/ml Lösung.
Die neuen Wirkstoffe können in üblicher Weise angewendet werden, insbesondere sind folgende Anwendungsmethoden als bevorzugt zu nennen :

a) parenteral : intravenös, intramuskulär, subkutan, intraartikulär, intratumoral
b) lokal : z. B. intranasal
c) oral

Als Dosierungsbereich kann für die neuen erfindungsgemäßen Wirkstoffe angegeben werden :
0,1-20 mg Wirkstoff/kg Körpergewicht, vorzugsweise 1 bis 10 mg Wirkstoff/kg Körpergewicht, die Dosierung ist dabei vor allem abhängig von der zu behandelnden Spezies sowie von der Applikationsart.
Die erfindungsgemäßen neuen Wirkstoffe können bei Mensch und Tier eingesetzt werden.

Beispiel 1

a) Isolierung von physiologischem Rinder-ITI und Abtrennung von BI-8$^+$.

Unter gutem Durchmischen wurden bei Raumtemperatur 428,6 g 70 % Perchlorsäure in 10 l Rinderserum eingerührt (257 ml) und die ausgefallenen Proteine nach zweistündigem Stehen abzentrifugiert (40 Min., 3 000 g). Das Sediment enthält die Hauptmasse des nativen und gegebenenfalls auch partiell abgebauten ITI und wurde, wie unter b) beschrieben, zur Gewinnung des inhibitorisch aktiven polyvalenten Inhibitors mit Mr 14 000 verwendet.
Das klare Zentrifugat wurde mit 5 N Kaliumhydroxidlösung (ca. 600 ml) neutralisiert und das ausgefallene Kaliumperchorat durch Filtration abgetrennt. Der Filtrationsrückstand wurde mit Wasser gewaschen bis das Filtrat bei 280 nm optisch leer war. Dann wurde der pH-Wert der Lösung mit 2 N Kaliumhydroxidlösung auf 7,8 eingestellt und unter langsamen mechanischem Rühren solange Trypsin-Sepharose 4B zugesetzt, bis die gesamte antitryptische Aktivität (56 inhibitorische Einheiten) gebunden war. Die mit den Trypsininhibitoren beladene Trypsin-Sepharose wurde durch Absaugen der Suspension über eine Fritte (D2) isoliert. Der Filtrationsrückstand wurde mit 500 ml 0,2 M Triethanolamin-Salzsäure Puffer pH 7,8, der 0,2 M an Natriumchlorid war, gewaschen und in 250 ml 0,2 M Kaliumchlorid-Salzsäurelösung pH 1,5 suspendiert. Man filtrierte die Suspension nach 2 Stunden Stehen bei 20 °C über eine Säule (5 × 30 cm), die mit der Kaliumchlorid-Salzsäurelösung eluiert wurde, bis das Eluat bei 280 nm keine Extinktion mehr hatte (Gesamtvolumen ca. 500 ml). Durch Zugabe von Natriumhydroxydlösung wurde der pH-Wert der Eluate auf 7,8 gestellt und ihr Volumen durch Ultrafiltration über eine Amicon UM-2-Membrane auf 25 ml reduziert. Man filtriert das Retentat über Sephadex G-75, fine (Säule 3 × 200 cm) mit 0,005 M Ammoniumacetatpuffer pH 7,5 als Elutionsmittel. Nach ihren antitryptischen Aktivitäten wurden die Eluate in drei Fraktionen unterteilt :

1. physiologischer ITI (BI-30) mit Mr 30 000 (ca. 5 inh. Einheiten ; Elutionsvolumen 450-600 ml)
2. modifizierter ITI (BI-14) mit Mr. 14 000 (ca. 20 inh. Einheiten ; Elutionsvolumen 600-795 ml)
3. Seruminhibitor (BI-8$^+$) mit Mr 8 000 (ca. 25 inh. Einheiten ; Elutionsvolumen 795-975 ml)

b) Gewinnung des polyvalenten Tandem-Inhibitors BI-14

Das nach a) isolierte Perchlorsäurepräzitpitat wurde in 7 l Wasser suspendiert. Die Suspension wurde mit 5 N Kaliumhydroxydlösung bis zum Erreichen von pH 8,0 versetzt und zum Homogenisieren 12 Stunden bei 20 °C gerührt. Dann wurde die ITI-Fraktion 1 der Sephadex G-75 Chromatographie zu der Suspension zugefügt und der pH-Wert des Gemisches mit N Natriumhydroxydlösung nachgestellt auf pH 8,0. Nach dem Erwärmen auf 37 °C wurden dem Reaktionsgemisch 3 g Rindertrypsin zugesetzt. Man hielt 1 Std. unter langsamen, mechanischem Rühren bei 37 °C. Dann wurden 325 g 70 % Perchlorsäure (193 ml) unter gutem Rühren zu der Mischung gegeben, und die entstandene Fällung wurde nach 2 Std. Stehen bei Raumtemperatur durch Abzentrifugieren (40 Min. ; 3 000 g) abgetrennt. Man neutralisierte das Zentrifugat durch Zugabe von 5 N Kaliumhydroxydlösung und entfernte das ausgefallene Kaliumperchlorat durch Filtration. Nun wurde der pH-Wert des Filtrats mit Kaliumhydroxydlösung auf 7,8 gebracht und unter langsamen Rühren so lange Trypsin-Sepharose portionsweise zugesetzt, bis die gesamte antitryptische Aktivität der Lösung (105 inh. Einheiten) an dem Affinitätsträger gebunden war. Man isolierte den immobilisierten Enzym-Inhibitor-Komplex, wie unter a) beschrieben, durch Absaugen über eine Glasfritte. Durch Filtration der Suspension der Trypsin-Sepharose in 0,2 M Kaliumchlorid-Salzsäurelösung pH 1,5 wurde, wie ebenfalls unter a) beschrieben, eine Lösung der säurestabilen Trypsininhibitoren erhalten. Diese Lösung wurde nach dem Neutralisieren mit 5 N Natriumhydroxydlösung durch Einengen im Vakuum auf ca. 50 ml konzentriert und über eine Sephadex G-75 Säule (3 × 200 cm) filtriert mit 0,01 M Ammoniumacetatpuffer als Elutionsmittel. Man engte die das BI-14 enthaltenden Eluate (Elutionsvolumen 600-800 ml bzw. Fraktion 40-54 bei einem Volumen von 15 ml/Fraktion) durch Ultrafiltration über eine Amicon UM-2-Membrane auf ein Volumen von 25 ml ein und entsalzte diese Lösung durch Filtration über eine Bio-Gel P-2 Säule (5 × 50 cm) mit Wasser als Elutionsmittel, wobei 2 Proteinpeaks mit Elutionsvolumina von 200-400 ml bzw. 450-560 ml eluiert wurden ; ein weiterer Peak wurde beim Nachwaschen der Säule mit einer mit Salzsäure auf pH 1,5 eingestellten 0,2 M Kaliumchloridlösung ausgewaschen, wenn der BI-8$^+$ nicht, wie unter a) beschrieben, zuvor durch Chromatographie an Sephadex G 75 abgetrennt worden war. Aus dem Peak I wurden durch Gefriertrocknen 100-110 mg BI-14 isoliert (50-55 % der eingesetzten antitryptischen Aktivität). Peak II und das Kaliumchlorideluat enthielten jeweils 35 mg eines Inhibitors, der nur Trypsin hemmt (jeweils 7 % der eingesetzten antitryptischen Aktivität). Das Hemmprofil von BI-14 ist aus der Tabelle 1 ersichtlich.

c) Isolierung des Elastase inhibitors BI-8-E

200 mg (14,3 μ Mole) nach b) erhaltenes BI-14 wurden in 75 ml 0,2 M Triethanolamin-Salzsäure Puffer pH 7,8 gelöst und mit 800 mg zugelöstem Trypsin (30 μ Mole) 2 Std. bei 37 °C inkubiert. Dann wurden 7,5 ml 70 % Perchlorsäure zu der Lösung hinzugefügt und das Präzipitat nach 6-stündigem Stehen durch Abzentrifugieren (30 Minuten, 5 000 g) abgetrennt. Das Zentrifugat wurde mit 5 N Kaliumhydroxydlösung neutralisiert (18 ml) und das ausgefallene Kaliumperchorat durch Filtration abgetrennt. Das Filtrat wurde unter Verwendung einer Amicon UM-2-Membrane durch Ultrafiltration auf ein Volumen von 20 ml konzentriert. Das Konzentrat wurde auf eine 3 × 200 cm Sephadex G-75 Säule aufgetragen und die Säule entwickelt mit 0,05 M Natrium-Boratpuffer pH 8,0 der 0,2 M Natriumchlorid enthielt.

In den Eluaten wurden die antitryptische Wirkung und die Elastaseaktivität bestimmt. Das Ergebnis der Chromatographie ist in Abb. 1 wiedergegeben. Das im 2. Peak neben dem BI-8-E eluierte BI-8-T wurde in der schon mehrfach beschriebenen Weise durch direkte Filtration der Lösung über eine 3 × 15 cm Trypsin-Sepharose-Säule abgetrennt. Die Durchläufe mit dem BI-8-E wurden durch Ultrafiltration mit einer UM-2 Amicon-Membrane auf ein Volumen von 20 ml eingeengt und diese Lösung wurde anschließend durch Filtration über eine mit einer 1 M Ammoniumacetatlösung äquilibrierte Bio-Gel P-2 Säule — 1,5 × 150 cm — entsalzt. Die den Inhibitor enthaltenden Fraktionen wurden vereinigt und das BI-8-E durch Gefriertrocknen isoliert. Man erhielt dabei 15 mg (~ 15 %) farbloses BI-8-E. Angaben zum Hemmverhalten dieses Inhibitors sind in Tab. 1 enthalten.

d) BI-8-E$^+$

10 mg nach c) enthaltenes BI-8-E wurden in 2 ml 80 % Ameisensäure gelöst. Diese Lösung wurde 12 Std. bei 56 °C gehalten. Dann wurde die Lösung über eine Bio-Gel P-2 Säule filtriert (1,5 × 100 cm) mit Wasser als Elutionsmittel. Die proteinenthaltenden Eluate wurden vereinigt und gefriergetrocknet. Man gewann dabei 4,5-6,5 mg farblose Substanz mit 50-80 % der eingesetzten elastaseinhibitorischen Wirksamkeit. Das Hemmspektrum des erfindungsgemäßen, kohlenhydratfreien BI-8-E$^+$ ist aus Tabelle 1 ersichtlich.

Beispiel 2

Aus 10 l Rinderserum nach Beispiel 1.a erhaltenes Perchlorsäurepräzipitat wurde mit 7 l Wasser angeteigt. Nach der Zugabe von 5 N Natriumhydroxydlösung bis pH 8,2 wurde durch 12-stündiges Rühren bei 20 °C homogenisiert. Der Suspension wurden 3,5 g Trypsin zugesetzt und das Gemisch 18 Std. unter langsamen Rühren bei 37 °C gehalten. Dann wurde 325 g 70 % Perchlorsäure (195 ml) zu der Mischung gegeben und nach zweistündigem Stehen bei 20 °C das Präzipitat durch Zentrifugation (40 Min ; 3 000 g) abgetrennt. Der pH-Wert des Zentrifugats wurde mit 5 N Kaliumhydroxydlösung auf 7,8 gestellt. Man trennte das ausgefallene Kaliumperchlorat durch Filtration ab. Durch portionsweise Zugabe von Trypsin-Sepharose 4 B wurden die im Filtrat vorhandenen Trypsininhibitoren komplexiert und die unlöslichen Enzym-Inhibitor-Komplexe durch Filtration abgetrennt. Man reduzierte das Volumen des Filtrats im Vakuum auf ca. 700 ml und filtrierte das Filtrat über eine mit 0,05 M Tris-Salzsäure Puffer pH 8,0 äquilibrierte und mit Concanavalin-A Sepharose gefüllte Säule (2,5 × 20 cm) mit dem Äquilibrierpuffer als Elutionsmittel. Der erfindungsgemäße Elastaseinhibitor BI-8 sowie modifizierter BI-14⁺ wurden eluiert mit 0,05 M Natriumacetatpuffer pH 6,0 der 0,05 M an α -Methylmannosid war. Die Inhibitor enthaltenden Eluate wurden durch Ultrafiltration über eine Amicon UM-2-Membrane entsalzt.

Die Trennung von BI-14⁺ und BI-8-E wurde, wie bei Beispiel 1.c beschrieben, durch Gelfiltration an Sephadex G-75 vorgenommen. Nach dem Entsalzen über Bio-Gel p-2 und Gefriertrocknen wurden ∼ 10 mg BI-8-E erhalten.

## Beispiel 3

200 mg nach Beispiel 1.b erhaltenes BI-14 wurden in 75 ml 0,2 M Triethanolamin-Salzsäure Pufer pH 7,8 gelöst und wie bei 1 c) beschrieben, mit 800 mg Trypsin gespalten und aufgearbeitet. Die nach dem Abtrennen des Kaliumperchlorates erhaltene Lösung der Inhibitoren wurde, wie bei Beispiel 1.a beschrieben, mit Trypsin-Sepharose 4B versetzt bis die gesamte antitryptische Aktivität der Lösung an das Trypsin gebunden war. Dann wurde die beladene Trypsin-Sepharose durch Filtration über eine Fritte (D 2) abgetrennt und gut mit insgesamt 200 ml 0,2 M Triethanolamin-Salzsäure Puffer pH 7,8, der 0,2 M an Natriumchlorid war, nachgewaschen. Filtrat und Waschwasser wurden vereinigt und mit Chymotrypsin-Sepharose 4 B versetzt, bis im Überstand keine Elastaseinhibition mehr nachweisbar war. Die mit dem BI-14⁺ und dem BI-8-E beladene Chymotrypsin-Sepharose wurde in eine 2,5 × 30 cm Säule eingefüllt und mit dem obigen Triethanolamin-Salzsäure Puffer gewaschen.

Zur Desorption der Elastaseinhibitoren BI-14⁺ und BI-8-E wurde die Säule nach dem Auswaschen mit Wasser mit 250 ml einer 0,2 M Kaliumchlorid-Salzsäurelösung pH 1,5 entwickelt, bis die Eluate keine Extinktion bei 280 nm aufwiesen. Die Eluate mit Elastasehemmaktivität wurden vereinigt, und das Volumen der so erhaltenen Lösung wurde nach dem Neutralisieren mit N Kaliumhydroxydlösung durch Ultrafiltration über eine Amicon UM-2-Membrane auf ca. 10 ml reduziert.

Zur Abtrennung von BI-14⁺ und der restlichen Salze wurde das Konzentrat über eine mit Sephadex G-75 gefüllte Säule — 3 × 200 cm — filtriert.

Als Elutionsmittel diente die 0,1 M Ammoniumacetatlösung pH 7,5.

Die Eluate wurden in Fraktionen zu je 24 ml aufgefangen und ihre Elastasehemmwirkung untersucht. Das Elutionsprofil der Säule ist aus Abb. 1 ersichtlich. Die den BI-8-E enthaltenden Eluate wurden vereinigt und der Inhibitor durch Gefriertrocknung isoliert. Man erhielt 10-20 mg farbloses Lyophilisat. Aus dem BI-8-E läßt sich der BI-8-E⁺ herstellen, wie bei Beispiel 1.d beschrieben.

(Siehe Tabelle 1 Seite 10 f.)

Tabelle 1

Hemmspektren der aus Rinder-ITI gewonnenen Inhibitoren

| INHIBITOR | E N Z Y M | | | | |
|---|---|---|---|---|---|
| | $\zeta$-Chymotryp-sin (Rind) | Granulozyten-elastase (Mensch) | Pankreas-elastase (Schwein) | Kathepsin G (Mensch) | Trypsin (Rind) |
| BI-14 | ++++ | +++ | +++ | ++ | +++ |
| BI-14$^+$ | +++ | +++ | +++ | ++ | (+) |
| BI-8-E (erfindungs-gemäß) | +++ | +++ | +++ | ++ | – |
| BI-8-E$^+$ (erfindungs-gemäß) | +++ | +++ | +++ | ++ | – |
| BI-8-T | ++ | – | – | ++ | +++ |

**Patentansprüche**

1. Elastaseinhibitoren der allgemeinen Formel I
Lys-Ala-Asp-Ser-Cys-Gln-Leu-Asp-Tyr-Ser-Gln-Gly-Pro-Cys-Leu-Gly-Leu-Phe-Lys-Arg-Tyr-Phe-Tyr-
X
Asn-Gly-Thr-Ser-Met-Ala-Cys-Glu-Thr-Phe-Leu-Tyr-Gly-Gly-Cys-Met-Gly-Asn-Leu-Asn-Asn-Phe-Leu-Ser-
Gln-Lys-Glu-Cys-Leu-Glu-Thr-Cys-Arg,

(I)

in der X für Wasserstoff oder einen Glycosidrest steht.

2. Elastaseinhibitoren der allgemeinen Formel (I) in Anspruch 1, in der X einen Glycosidrest bedeutet (BI-8-E).

3. Elastaseinhibitor der allgemeinen Formel (I) in Anspruch 1, in der X Wasserstoff bedeutet (BI-8-E$^+$).

4. Verfahren zur Herstellung von Elastaseinhibitoren der allgemeinen Formel (I) in Anspruch 1, dadurch gekennzeichnet, daß man den Inter-α-trypsininhibitor vom Rind oder die aus diesem Inhibitor durch Spaltung erhaltenen Inhibitoren BI-30 oder BI-14 oder Gemische derselben mit einem in einem Überschuß von 10 bis 400 %, vorzugsweise 150 bis 300 %, anzuwendenden proteolytischen Enzym bei pH-Werten von 6,5 bis 10 1 bis 24 Stunden, vorzugsweise 1 bis 10 Stunden, bei etwa 37 °C behandelt und den dabei entstehenden Inhibitor isoliert und gegebenenfalls durch Behandeln mit Säure den BI-8-E$^+$ überführt.

5. Verfahren gemäß Anspruch 4, dadurch gekennzeichnet, daß man als proteolystisches Enzym Trypsin verwendet.

6. Verfahren gemäß Anspruch 4, dadurch gekennzeichnet, daß man als Säure 60-90 %ige Ameisensäure bei Temperaturen von 40-70 °C, vorzugsweise 56 °C verwendet.

7. Arzneimittel, gekennzeichnet durch einen Gehalt an einem Elastaseinhibitor der allgemeinen Formel (I) in Anspruch 1.

8. Verwendung von Elastaseinhibitoren gemäß den Ansprüchen 1 bis 3 zur Herstellung von Arzneimitteln gegen Schocks.

9. Verwendung von Elastaseinhibitoren gemäß den Ansprüchen 1 bis 3 zur Herstellung von Arzneimitteln gegen rheumatische Arthritis.

10. Verwendung von Elastaseinhibitoren gemäß den Ansprüchen 1 bis 3 zur Herstellung von Arzneimitteln gegen Lungenemphysem.


**Claims**

1. Elastase inhibitors of the general formula I
Lys-Ala-Asp-Ser-Cys-Gln-Leu-Asp-Tyr-Ser-Gln-Gly-Pro-Cys-Leu-Gly-Leu-Phe-Lys-Arg-Tyr-Phe-Tyr-
X
Asn-Gly-Thr-Ser-Met-Ala-Cys-Glu-Thr-Phe-Leu-Tyr-Gly-Gly-Cys-Met-Gly-Asn-Leu-Asn-Asn-Phe-Leu-Ser-
Gln-Lys-Glu-Cys-Leu-Glu-Thr-Cys-Arg,

(I)

in which X represents hydrogen or a glycoside residue.

2. Elastase inhibitors of the general formula (I) in Claim 1, in which X denotes a glycoside residue (BI-8-E).

3. Elastase inhibitor of the general formula (I) in Claim 1, in which X denotes hydrogen (BI-8-E$^+$).

4. Process for the preparation of elastase inhibitors of the general formula (I) in Claim 1, characterised in that the inter-α-trypsin inhibitor from cattle or the inhibitors BI-30 or BI-14 obtained from this inhibitor by cleavage, or mixtures thereof, are treated with a proteolytic enzyme, which is to be used in an excess of 10-400 %, preferably 150-300 %, at pH values of 6.5-10 for 1 to 24 hours, preferably 1 to 10 hours, at about 37 °C, and the inhibitor thereby formed is isolated and, if desired converted into the BI-8-E$^+$ by treating it with an acid.

5. Process according to Claim 4, characterised in that trypsin is used as the proteolytic enzyme.

6. Process according to Claim 4, characterised in that 60-90 % formic acid is used as the acid, at temperatures of 40-70 °C, preferably 56 °C.

7. Medicaments, characterised in that they contain an elastase inhibitor of the general formula (I) in Claim 1.

8. Use of elastase inhibitors according to Claims 1 to 3 for the preparation of medicaments against shocks.

9. Use of elastase inhibitors according to Claims 1 to 3 for the preparation of medicaments against rheumatoid arthritis.

10. Use of elastase inhibitors according to Claims 1 to 3 for preparing medicaments against pulmonary emphysema.

**Revendications** .

1. Inhibiteurs d'élastases répondant à la formule générale I.
Lys-Ala-Asp-Ser-Cys-Gln-Leu-Asp-Tyr-Ser-Gln-Gly-Pro-Cys-Leu-Gly-Leu-Phe-Lys-Arg-Tyr-Phe-Tyr-
X
Asn-Gly-Thr-Ser-Met-Ala-Cys-Glu-Thr-Phe-Leu-Tyr-Gly-Gly-Cys-Met-Gly-Asn-Leu-Asn-Asn-Phe-Leu-Ser-
Gln-Lys-Glu-Cys-Leu-Glu-Thr-Cys-Arg,

(I)

dans laquelle X représente l'hydrogène ou un reste glucoside.

2. Inhibiteurs d'élastases répondant à la formule générale (I) de la revendication 1, dans laquelle X représente un reste glucoside (BI-8-E).

3. Inhibiteurs d'élastases répondant à la formule générale (I) de la revendication 1, dans laquelle X représente l'hydrogène (BI-8-E⁺).

4. Procédé pour la préparation d'inhibiteurs d'élastases répondant à la formule générale (I) de la revendication 1, caractérisé en ce que l'on traite l'inhibiteur d'inter-α-trypsine de génisse, les inhibiteurs BI-30 ou BI-14 obtenus par scission de cet inhibiteur ou leurs mélanges pendant 1 à 24 heures, de préférence 1 à 10 heures à environ 37 °C et à des valeurs de pH de 6,5 à 10, à l'aide d'une enzyme protéolytique devant être mise en œuvre avec un excès de 10 à 400 %, de préférence de 150 à 300 %, que l'on isole l'inhibiteur ainsi obtenu et le transforme, le cas échéant, en BI-8-E⁺ par traitement avec un acide.

5. Procédé selon la revendication 4, caractérisé en ce que l'on emploie de la trypsine comme enzyme protéolytique.

6. Procédé selon la revendication 4, caractérisé en ce que l'on emploie, en tant qu'acide, de l'acide formique à 60-80 % à des températures de 40 à 70 °C, de préférence à 56 °C.

7. Médicaments caractérisés en ce qu'ils contiennent un inhibiteur d'élastase répondant à la formule générale (I) de la revendication 1.

8. Utilisation d'inhibiteurs d'élastases selon les revendications 1 à 3 pour la préparation de médicaments contre le choc.

9. Utilisation d'inhibiteurs d'élastases selon les revendications 1 à 3 pour la préparation de médicaments contre l'arthrite rhumatoïde.

10. Utilisation d'inhibiteurs d'élastases selon les revendications 1 à 3 pour la préparation de médicaments contre l'emphysème pulmonaire.

FIG.1

FIG. 2

FIG. 3